(19) Europäisches Patentamt
European Patent Office
Office européen des brevets

(11) EP 3 087 898 A1

(12) **EUROPEAN PATENT APPLICATION**
published in accordance with Art. 153(4) EPC

(43) Date of publication:
02.11.2016 Bulletin 2016/44

(51) Int Cl.:
A61B 1/00 (2006.01)          G02B 23/24 (2006.01)

(21) Application number: 14874023.6

(22) Date of filing: 09.10.2014

(86) International application number:
PCT/JP2014/077044

(87) International publication number:
WO 2015/098236 (02.07.2015 Gazette 2015/26)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 24.12.2013 JP 2013265326

(71) Applicant: Olympus Corporation
Tokyo 192-8507 (JP)

(72) Inventor: NISHIJIMA Yoshikazu
Hachioji-shi
Tokyo 192-8507 (JP)

(74) Representative: Schicker, Silvia
Wuesthoff & Wuesthoff
Patentanwälte PartG mbB
Schweigerstraße 2
81541 München (DE)

(54) **ENDOSCOPE**

(57)     An endoscope 2 includes a rigid distal end portion 7 provided in a distal end portion of an insertion section 4, a bending portion 8, provided on a proximal end side of the rigid distal end portion 7 of the insertion section 4, and configured to bend according to a bending operation by an operation section 5, a flexible tube portion 10 provided on a proximal end side of the bending portion 8, and an elastic cylindrical portion 9, provided between the bending portion 8 and the flexible tube portion 10, and configured not to bend according to the bending operation by the operation section 5, the elastic cylindrical portion 9 having a rigidity to bending higher than a rigidity to bending of the bending portion 8 and lower than a rigidity to bending of the flexible tube portion 10.

## FIG. 2

## Description

Technical Field

The present invention relates to an endoscope, and more particularly, to an endoscope having a bending portion on the distal end side of an insertion section.

Background Art

Conventionally, endoscopes are widely used in industrial and medical fields. An endoscope has an elongated insertion section, and a rigid distal end portion having an observation window and an illumination window is provided at a distal end portion of the insertion section.

A bending portion is provided on the proximal end side of the rigid distal end portion of the insertion section. The bending portion of the endoscope may be bent in two directions of up and down or left and right, or in four directions of up, down, left, and right. When a bending operation member of an operation section is operated, a bending wire inserted through the insertion section is pulled or relaxed according to the operation, thereby bending the bending portion. Accordingly, a user, who is an examining person, may operate an operation member, such as a bending operation knob, of the operation section connected to a proximal end portion of the insertion section to thereby bend the bending portion and to bend the distal end of the insertion section in a desired direction, and may perform insertion operation of the insertion section, observation of an examination target, and the like.

Inside the examination target into which the insertion section of the endoscope is to be inserted, there are parts which are curved greatly, such as a tubular tract with small curvature, parts where the bending portion cannot be inserted further unless it is greatly bent, and the like. At such parts, the user deliberately performs insertion operation and the like of the insertion section while bending the bending portion.

Also, for example, Japanese Utility Model Publication No. 1-22641 proposes an endoscope having a first bending portion on the distal end side of a bending portion and a second bending portion on the proximal end side so as to enable smooth insertion of an insertion section in a case where the route in an examination target where the insertion section is to be inserted is greatly curved.

However, even if the examining person operates the insertion section deliberately, the distal end portion of the insertion section advances inside the examination target while running into a wall or the like inside the examination target.

Rigidity to bending of the bending portion is lower than the rigidity to bending of a flexible tube portion. In other words, the bending portion is a soft portion which bends with substantially no resistance in four directions, and the flexible tube portion bends in four directions but with some resistance, and is less easily bent than the bending portion.

When the insertion section is inserted in an examination target, if the insertion section is pushed in strongly after the rigid distal end portion abuts against a wall or the like, the bending portion is immediately bent because its rigidity to bending is low. However, the flexible tube portion with higher rigidity to bending than the bending portion is not easily bent, and thus the bending portion may become sharply bent.

When the bending portion is sharply bent, a great bending stress is applied to the connection part, and a component, such as a cable, inserted through the insertion section may be fractured, and the insertion section may be broken. Also, in the industrial field, the outer skin of the insertion section may be torn by being scraped strongly inside a metal pipe.

Moreover, when the bending portion is sharply bent, the insertion performance of the insertion section is reduced.

Accordingly, the present invention has its object to provide an endoscope whose insertion section has high insertion performance, by preventing a bending portion of the insertion section from being sharply bent and making the insertion section less prone to break.

Disclosure of Invention

Means for Solving the Problem

An endoscope of an aspect of the present invention includes a rigid distal end portion provided in a distal end portion of an insertion section, a bending portion, provided on a proximal end side of the rigid distal end portion of the insertion section, and configured to bend according to a bending operation by an operation section, a flexible tube portion provided on a proximal end side of the bending portion, and an elastic cylindrical portion provided between the bending portion and the flexible tube portion, where the elastic cylindrical portion does not bend according to the bending operation by the operation section, and has a rigidity to bending higher than a rigidity to bending of the bending portion and lower than a rigidity to bending of the flexible tube portion.

Brief Description of the Drawings

Fig. 1 is a configuration diagram showing a configuration of an endoscope device according to a first embodiment of the present invention;
Fig. 2 is a cross-sectional diagram of an insertion section according to the first embodiment of the present invention;
Fig. 3 is a diagram for describing a state where an insertion section 101 of an endoscope not including an elastic cylindrical portion 9 has started advancing along a greatly curved inner wall W inside an examination target;

Fig. 4 is a diagram for describing a state where the insertion section 101 of the endoscope not including the elastic cylindrical portion 9 has further advanced along the greatly curved inner wall inside the examination target;

Fig. 5 is a diagram for describing a state where an insertion section 4 of an endoscope 2 according to the first embodiment of the present invention has started advancing along a greatly curved inner wall W inside an examination target;

Fig. 6 is a diagram for describing a state where the insertion section 4 of the endoscope 2 according to the first embodiment of the present invention has further advanced along the greatly curved inner wall inside the examination target;

Fig. 7 is a cross-sectional diagram of an insertion section according to a second embodiment of the present invention;

Fig. 8 is a schematic cross-sectional diagram for describing a configuration of an elastic cylindrical portion 9B, according to the second embodiment of the present invention, having a tightly wound coil which is formed by using a wire whose wire diameter is gradually changed, the diagram showing the elastic cylindrical portion 9B along the axial direction; and

Fig. 9 is a schematic cross-sectional diagram for describing a configuration of an elastic cylindrical portion having a rubber member, according to an example modification of the first and the second embodiments, the diagram showing an elastic cylindrical portion 9C along the axial direction.

Best Mode for Carrying Out the Invention

Hereinafter, embodiments of the present invention will be described with reference to the drawings.

Note that, in each of the drawings used in the following description, the scale of display of each component is made different such that each component is large enough to be recognized in the drawing. Accordingly, the present invention is not restricted to the modes shown in the drawings with respect to the number of components, the shapes of the components, the proportion of the sizes of the components, and the relative positional relationship of respective components.

(First Embodiment)

(Configuration of Endoscope Device)

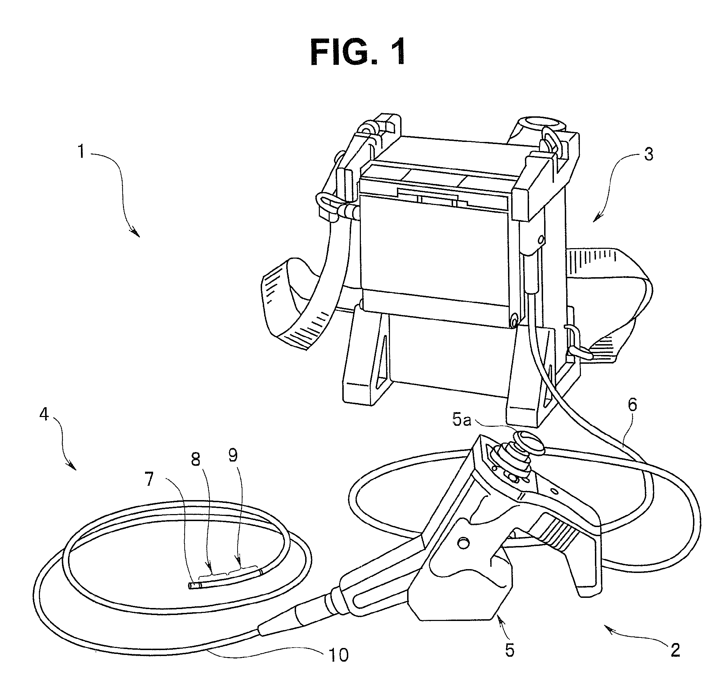

Fig. 1 is a configuration diagram showing a configuration of an endoscope device according to a present embodiment.

As shown in Fig. 1, an endoscope system 1 is configured by including an endoscope 2, and a device main body 3 connected to the endoscope 2.

The endoscope 2 is configured by including a flexible, elongated insertion section 4, an operation section 5 connected to a proximal end portion of the insertion section 4, and a universal cord 6 extending from the operation section 5.

The insertion section 4 has a rigid distal end portion 7, a bending portion 8, an elastic cylindrical portion 9, and a long flexible tube portion 10 continuously provided in this order from the distal end side of the insertion section 4, and the operation section 5 is connected to a proximal end portion of the flexible tube portion 10.

The bending portion 8 is capable of being bent in, for example, four directions of up, down, left, and right by operation of a joystick 5a provided at the operation section 5. Note that, in addition to the joystick 5a, the operation section 5 is provided with various switches and the like such as for instructing an image pickup device (not shown) provided inside the rigid distal end portion 7 to perform a photographing operation.

The device main body 3 is box-shaped, for example, and is provided, on the outer housing, with a monitor configured to display an endoscopic image captured by the endoscope 2.

(Configuration of Insertion Section)

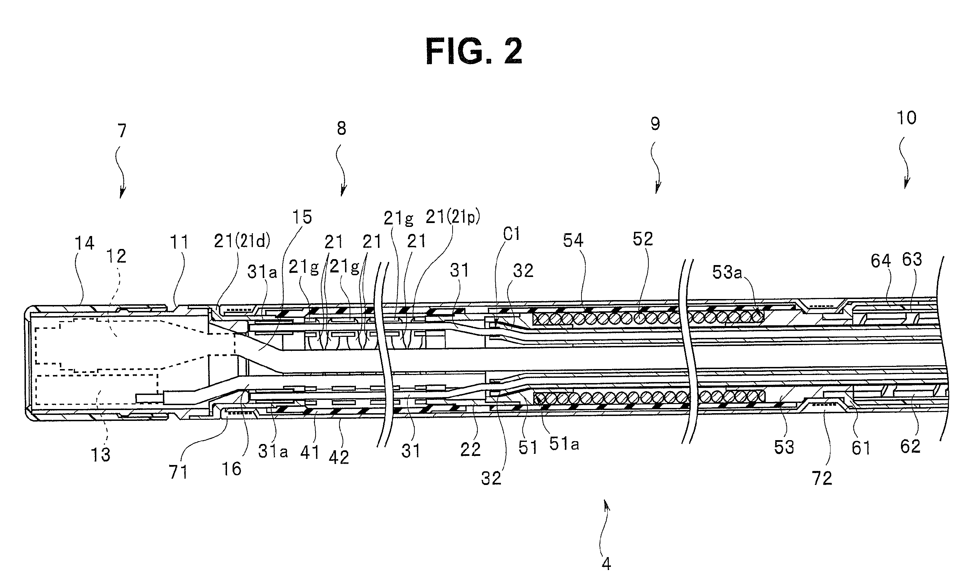

Fig. 2 is a cross-sectional diagram of the insertion section according to the present embodiment.

The rigid distal end portion 7 provided at the distal end portion of the insertion section 4 is configured by including a rigid distal end member 11 which is made of metal or resin and which has a columnar shape. An image pickup unit 12 and an illumination unit 13, shown by dotted lines, are provided inside the rigid distal end member 11. The image pickup unit 12 includes an imaging device, such as a CCD, configured to receive reflected light from an observation target, and the illumination unit 13 includes a light emitting device, such as a light emitting diode (LED), configured to emit illumination light. A cover member 14 covers the distal end side of the rigid distal end member 11.

An observation window and an illumination window are provided on a distal end surface of the rigid distal end member 11, and light passing through the observation window is received by the image pickup unit 12, and illumination light from the illumination unit 13 is emitted through the illumination window. The image pickup unit 12 outputs an image pickup signal to an image pickup signal cable 15 extending from a proximal end portion. The illumination unit 13 receives an illumination driving signal through a driving signal cable 16 extending from a proximal end portion.

The bending portion 8, which is provided on the proximal end side of the rigid distal end portion 7 of the insertion section 4 and which is bent according to the bending operation by the operation section, is configured from a plurality of annular bending pieces 21. Two adjacent bending pieces 21 are pivotally supported at two points in the radial direction, and the plurality of bending pieces 21 are configured to be capable of bending in the up, down, left and right directions. A proximal end portion of the rigid distal end portion 7 is fixed to a bending piece 21d, at the most distal end, of the bending portion 8 by a screw or the like.

A bending piece 21 p, at the most proximal end, of the bending portion 8 is connected to a part, on the distal end side, of a cylindrical joint receiving member 22.

A plurality of guide members 21 g having a circular cylindrical shape and fixed to the plurality of bending pieces 21 are disposed inside the bending portion 8, along the axial direction of the bending portion 8. A distal end portion of each bending wire 31 is crimped while being covered with a cylindrical member 31a, and is fixed to the bending piece 21 d at the most distal end. Four bending wires 31 are disposed through holes of the plurality of guide members 21 g, around the axis of the insertion section 4 and separated from one another by 90 degrees.

The outer circumferential surface of the bending portion 8 is covered with an elastic rubber tube 41. The rubber tube 41 is a member which makes the bending portion 8 waterproof. A thread (not shown) is wound around a part of the rubber tube 41, on the distal end side, and the part is applied with an adhesive and is fixed by being inserted over an outer circumferential portion of the bending piece 21 d at the most distal end. A thread (not shown) is wound around a part of the rubber tube 41, on the proximal end side, and the part is applied with an adhesive and is fixed by being inserted over an outer circumferential portion of the joint receiving member 22.

Furthermore, a tubular mesh tube 42 formed by knitting fine metal wires into a cylindrical shape covers over the outer circumferential surface of the bending portion 8. The mesh tube 42, which is a so-called metal braid, covers not only the bending portion 8, but also the elastic cylindrical portion 9 and the flexible tube portion 10.

Moreover, the plurality of contiguous bending pieces 21 are cylindrical, and the image pickup signal cable 15 and the driving signal cable 16 are inserted through a hollow portion inside the plurality of bending pieces 21.

A coil receiving member 51 on the distal end side is connected and is fixed by, for example, an adhesive or by welding at a part of the cylindrical joint receiving member 22, on the proximal end side. The coil receiving member 51 has a cylindrical shape, and a part of the coil receiving member 51, on the distal end side, is inserted inside the part of the joint receiving member 22, on the proximal end side.

Each bending wire 31 is inserted through a coil pipe 32. A distal end portion of each coil pipe 32 is fixed to a proximal end portion of the bending portion 8. The distal end portion of each coil pipe 32 is fixed to the coil receiving member 51 at a point C1 in Fig. 2 by an adhesive or by welding, for example.

That is, the distal end portion of each bending wire 31 is fixed to the bending piece 21d at the most distal end, and the distal end portion of each coil pipe 32 is fixed to the coil receiving member 51. Accordingly, when an examining person tilts the joystick 5a in a desired direction, the bending wire 31 corresponding to the direction is pulled or relaxed, and the bending portion 8 is bent.

A step portion 51a is formed at the outer circumferential portion of a part of the coil receiving member 51, on the proximal end side, along the circumferential direction. A distal end portion of a tightly wound coil 52 is connected to the proximal end portion of the coil receiving member 51 by being inserted over the outer cylindrical surface formed by the step portion 51a, and is fixed by an adhesive, by welding or soldering, for example. The tightly wound coil 52 is an elastic cylindrical member made of metal, such as stainless steel.

A proximal end portion of the tightly wound coil 52 is connected to a coil receiving member 53 on the proximal end side, and is fixed by an adhesive or by welding, for example. More specifically, a step portion 53a is formed at the outer circumferential portion of a part of the coil receiving member 53, on the distal end side, along the circumferential direction. The proximal end portion of the tightly wound coil 52 is connected to a distal end portion of the coil receiving member 53 on the proximal end side by being inserted over the outer cylindrical surface formed by the step portion 53a, and is fixed by an adhesive, or by welding or soldering, for example.

That is, the coil receiving members 51 and 53 have a cylindrical shape, and the distal end portion of the tightly wound coil 52 is fitted with the step portion 51a formed on the proximal end side of the coil receiving member 51, and the proximal end portion of the tightly wound coil 52 is fitted with the step portion 53a formed on the distal end side of the coil receiving member 53. An elastic rubber tube 54 covers the outer circumferential surfaces of the two coil receiving members 51, 53 and the tightly wound coil 52. The rubber tube 54 is a member which makes the elastic cylindrical portion 9 waterproof. A part of the rubber tube 54, on the distal end side, is wound with a thread (not shown) and applied with an adhesive, and is fixed by being connected to the outer circumferential portion of the joint receiving member 22. A part of the rubber tube 54, on the proximal end side, is wound with a thread (not shown) and applied with an adhesive, and is fixed by being connected to an outer circumferential portion of the coil receiving member 53.

As described above, the elastic cylindrical portion 9 is configured by the tightly wound coil 52 which is not compressed in the axial direction. Accordingly, the length of the tightly wound coil 52 in the axial direction of the elastic cylindrical portion 9 is not reduced in the axial direction of the insertion section 4.

A proximal end portion of the coil receiving member 53 is connected to a circular cylindrical fixing member 61, and is fixed by welding or the like. A distal end portion of the flexible tube portion 10 is connected to the proximal end side of the fixing member 61, and is fixed by an adhesive or the like.

The flexible tube portion 10 provided on the proximal end side of the elastic cylindrical portion 9 has a metal strip flex 62 wound around the outer circumference of the image pickup signal cable 15 and the like. The outer circumferential portion of the flex 62 is covered with a metal mesh member 63 having a cylindrical shape. The outer circumferential surface of the mesh member 63 is covered with a resin member 64.

As described above, the mesh tube 42 covers from the bending portion 8 to the flexible tube portion 10. A distal end portion of the mesh tube 42 is fixed by thread-winding to the outer circumferential portion of the bending piece 21d at the most distal end, and an adhesive 71 is applied to the part fixed by thread-winding. A thread is also wound around the outer circumferential portion of the mesh tube 42, at the part where the elastic cylindrical portion 9 and the flexible tube portion 10 are joined, and an adhesive 72 is applied to the part.

When comparing the rigidities to bending of the bending portion 8, the elastic cylindrical portion 9, and the flexible tube portion 10, a rigidity R1 to bending of the bending portion 8 which is not bent is lower than a rigidity R2 to bending of the elastic cylindrical portion 9. Also, the rigidity R2 to bending of the elastic cylindrical portion 9 is lower than a rigidity R3 of the flexible tube portion 10. That is, the relationship of R1, R2, and R3 satisfies the following expression (1).

$$R1 < R2 < R3 \quad \ldots (1)$$

In other words, the bending portion 8 when the bending wire 31 is not being pulled is easily bent than the elastic cylindrical portion 9 with respect to bending in four directions (up, down, left, and right directions). Also, the elastic cylindrical portion 9 is more easily bent than the flexible tube portion 10 with respect to bending in the four directions (up, down, left, and right directions).

Note that, when the length of the bending portion 8 in the axial direction of the insertion section 4 is given as L1 and the length of the elastic cylindrical portion 9 in the axial direction of the insertion section 4 is given as L2, L1 and L2 desirably satisfy the following expression (2).

$$L1 \le L2 \quad \ldots (2)$$

Note that, in the present case, the length L1 of the elastic cylindrical portion 9 and the length L2 of the bending portion 8 are the same.

As described above, the elastic cylindrical portion 9 is provided between the bending portion 8 and the flexible tube portion 10, is not bent according to the bending operation by the operation section, and also has a rigidity to bending higher than the rigidity to bending of the bending portion 8 and lower than the rigidity to bending of the flexible tube portion 10.

(Action)

Next, an action of the insertion section 4 of the present embodiment in a case where the insertion section 4 of the endoscope 2 advances along a greatly curved inner wall inside an examination target will be described.

First, a case will be described where an insertion section of an endoscope not including the elastic cylindrical portion 9 described above is pushed in along a greatly curved inner wall inside an examination target.

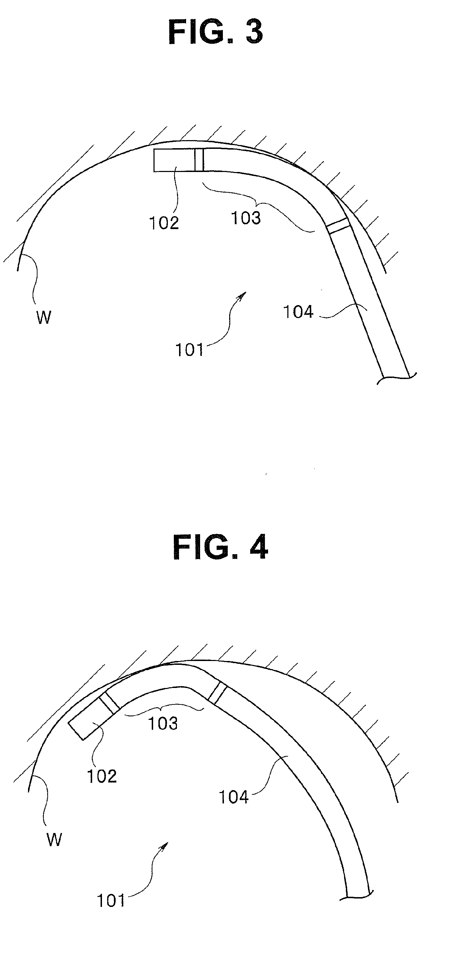

Fig. 3 is a diagram for describing a state where an insertion section 101 of an endoscope not including the elastic cylindrical portion 9 has started advancing along a greatly curved inner wall W inside an examination target. Fig. 4 is a diagram for describing a state where the insertion section 101 of the endoscope not including the elastic cylindrical portion 9 has further advanced along the greatly curved inner wall inside the examination target.

The insertion section 101 includes a bending portion 103 on the proximal end side of a rigid distal end portion 102, and includes a flexible tube portion 104 on the proximal end side of the bending portion 103. The rigid distal end portion 102 corresponds to the above-described rigid distal end portion 7, the bending portion 103 to the above-described bending portion 8, and the flexible tube portion 104 to the above-described flexible tube portion 10.

As shown in Fig. 3, the radius of curvature of the bending portion 103 is large when the rigid distal end portion 102 of the insertion section 101 starts advancing along the inner wall W, and the bending portion 103 is not greatly bent.

However, as shown in Fig. 4, when the insertion section 101 further advances along the greatly curved inner wall W inside the examination target, the bending portion 103 becomes sharply bent.

When the bending portion 103 is sharply bent, a great bending stress is applied to the bent part in a concentrated manner. This results in the fracture of a component, such as a cable, inserted through the insertion section 101, and the insertion section may be broken, or in the case where the inner wall W is an inner wall of a metal pipe, the outer skin of the insertion section 101 may be torn by being scraped strongly. Furthermore, when the bending portion 103 is sharply bent, the insertion performance of the insertion section 101 thereafter is reduced.

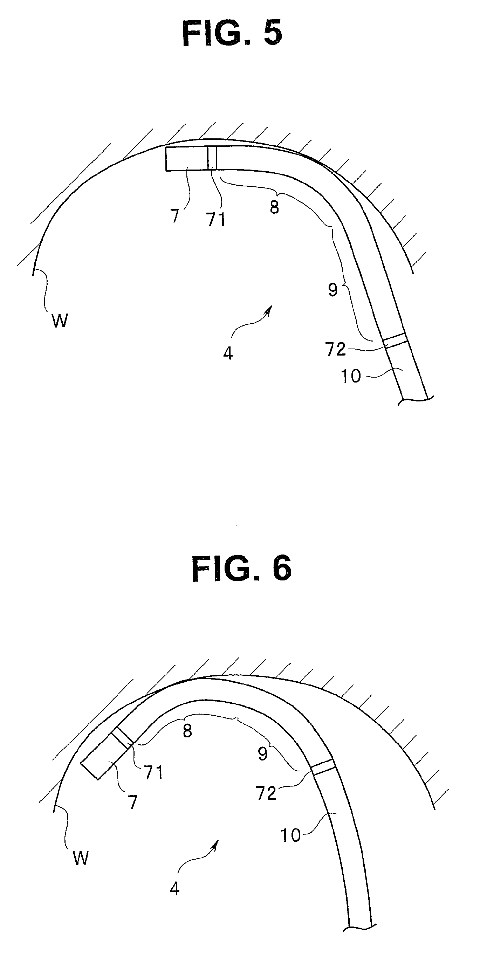

Fig. 5 is a diagram for describing a state where the insertion section 4 of the endoscope 2 according to the present embodiment has started advancing along a greatly curved inner wall W inside an examination target. Fig. 6 is a diagram for describing a state where the insertion section 4 of the endoscope 2 according to the present embodiment has further advanced along the greatly curved inner wall inside the examination target.

As shown in Fig. 5, the radius of curvature of the bending portion 8 is large when the rigid distal end portion 7 of the insertion section 4 starts advancing along the inner wall W, and the bending portion 8 is not greatly curved.

However, as shown in Fig. 6, when the insertion section 4 further advances along the greatly curved inner wall W inside the examination target, the elastic cylindrical portion 9 is bent successively to the bending portion 8, and the force of pushing is applied not only to the bending portion 8 but also to the elastic cylindrical portion 9. Accordingly, the bending portion 8 and the elastic cylindrical portion 9 are bent with larger radii of curvature than the radius of curvature of the bending portion 103 in Fig. 4.

Accordingly, since the elastic cylindrical portion 9 acts as a buffer portion configured to receive the force of pushing in in a dispersed manner when the insertion section 4 advances along the greatly curved inner wall W inside the examination target, and the bending portion 8 is not sharply bent, breaking of the insertion section due to the fracture of a component, such as a cable, inserted through the insertion section 4 may be prevented, and also, in the case where the inner wall W is an inner wall of a metal pipe, the outer skin of the insertion section 4 may be prevented from being torn by being scraped strongly, and furthermore, the insertion performance of the bending portion 8 is not reduced.

As described above, according to the present embodiment, an endoscope having the insertion section 4 with high insertion performance may be provided by making the insertion section 4 less prone to break by preventing the bending portion 8 of the insertion section 4 from being sharply bent.

(Second Embodiment)

The elastic cylindrical portion of the endoscope of the first embodiment has the same rigidity to bending across the entire length in the axial direction, but in a second embodiment, the rigidity to bending of a first flexible tube portion is not uniform along the axial direction of an insertion section.

In the following, the present embodiment will be described. The endoscope of the present embodiment has approximately the same configuration as the endoscope of the first embodiment, and thus the same components will be denoted with the same reference signs and description thereof will be omitted, and description will be given on only different components.

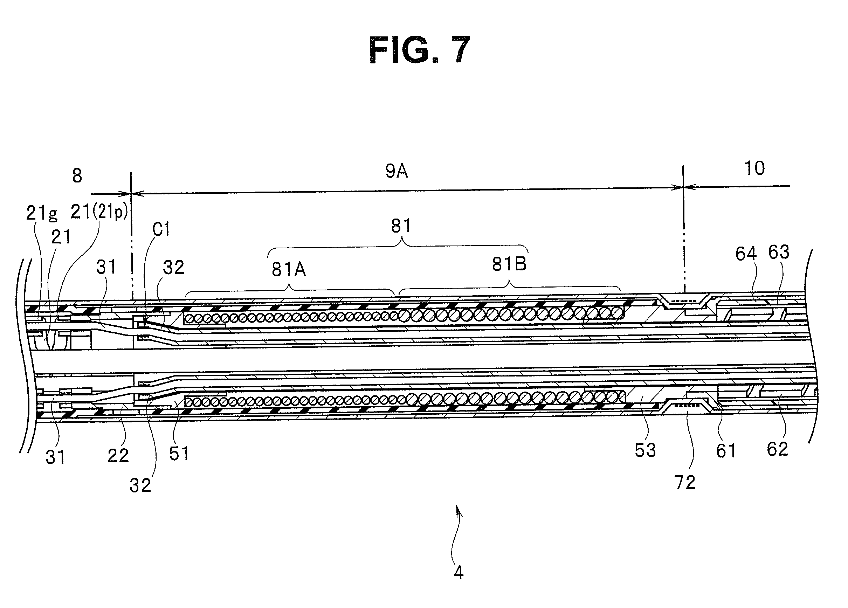

Fig. 7 is a cross-sectional diagram of an insertion section according to the present embodiment. An elastic cylindrical portion 9A is disposed between the bending portion 8 and the flexible tube portion 10. The elastic cylindrical portion 9A includes a tightly wound coil 81.

The tightly wound coil 81 is formed from two tightly wound coils 81A and 81B formed by wires with different wire diameters. The tightly wound coil 81A is disposed on the distal end side of the elastic cylindrical portion 9A, and the tightly wound coil 81B is disposed on the proximal end side of the elastic cylindrical portion 9A.

A proximal end portion of the tightly wound coil 81A and a distal end portion of the tightly wound coil 81B are fixed by being connected by welding or the like. Accordingly, the rigidity to bending of the elastic cylindrical portion 9A is not uniform along the axial direction of the insertion section 4. In the present case, the rigidity to bending of the elastic cylindrical portion 9A increases stepwise along the direction from the distal end portion toward the proximal end portion of the insertion section 4.

A rigidity R21 to bending at the part of the tightly wound coil 81 A of the elastic cylindrical portion 9A is lower than a rigidity R22 to bending at the part of the tightly wound coil 81B of the elastic cylindrical portion 9A.

Accordingly, the relationship of the rigidity R1 to bending of the bending portion 8, when the bending portion 8 is not bent, the rigidity R21 to bending at the part of the tightly wound coil 81 A of the elastic cylindrical portion 9A, the rigidity R22 to bending at the part of the tightly wound coil 81B of the elastic cylindrical portion 9A, and the rigidity R3 of the flexible tube portion 10 satisfies the following expression (3).

$$R1 < R21 < R22 < R3 \qquad ... (3)$$

In other words, according to the elastic cylindrical portion 9A, the part of the tightly wound coil 81 A of the elastic cylindrical portion 9A is more easily bent than the part of the tightly wound coil 81 B of the elastic cylindrical portion 9A.

According to the endoscope having such a configuration, the same effect as in the first embodiment may be achieved, and also, the elastic cylindrical portion 9A may be bent more gradually.

Note that, in the example described above, two tightly wound coils with different wire diameters are used for the elastic cylindrical portion 9A, but it is also possible to use three or more tightly wound coils with different wire diameters. That is, a plurality of tightly wound coils may be used, whose wire diameters increase from the distal end side of the elastic cylindrical portion 9A toward the proximal end side with the tightly wound coil with the smallest wire diameter disposed on the distal end side of the elastic cylindrical portion 9A and the tightly wound coil with the largest wire diameter disposed on the proximal end side of the elastic cylindrical portion 9A.

That is, according to the elastic cylindrical portion 9A shown in Fig. 7, the rigidity to bending is changed once along the elastic cylindrical portion 9A, but it is possible to use three or more tightly wound coils with different wire diameters. Here, the rigidity to bending is discretely changed at several positions along the elastic cylindrical portion 9A.

Further note that a tightly wound coil which is formed by using a wire whose wire diameter is gradually changed may be used for the elastic cylindrical portion 9A.

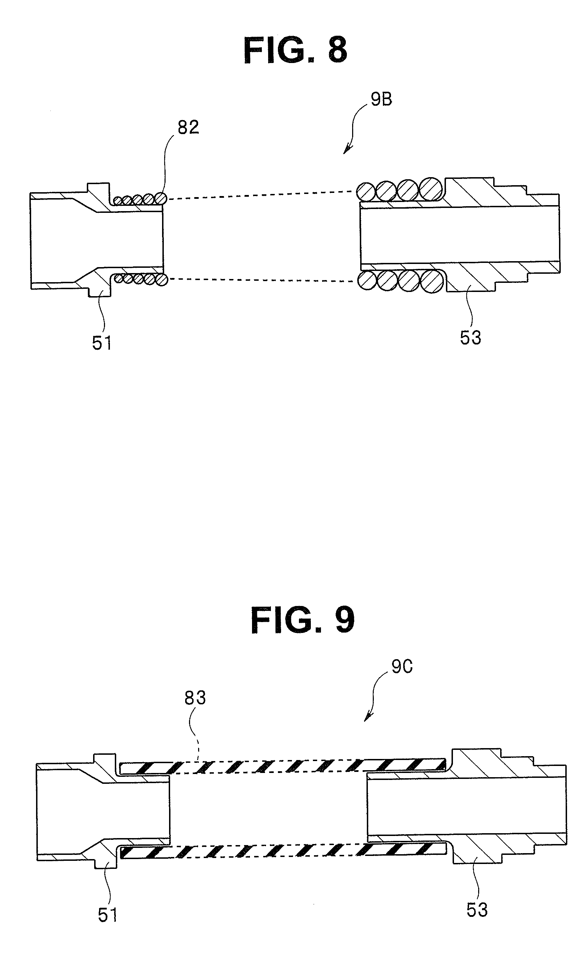

Fig. 8 is a schematic cross-sectional diagram for describing a configuration of an elastic cylindrical portion 9B having a tightly wound coil which is formed by using a wire whose wire diameter is gradually changed, the diagram showing the elastic cylindrical portion 9B along the axial direction.

A tightly wound coil 82 of the elastic cylindrical portion 9B is formed by using a wire whose wire diameter is gradually changed. As shown in Fig. 8, the tightly wound coil 82 is disposed between a proximal end portion of the coil receiving member 51 and a distal end portion of the coil receiving member 53, with the wire diameter increasing from the distal end side of the tightly wound coil 82 toward the proximal end side.

When the tightly wound coil 82, as shown in Fig. 8, formed by using a wire whose wire diameter is gradually changed is used, the rigidity to bending of the elastic cylindrical portion 9A continuously changes from the distal end side of the elastic cylindrical portion 9A toward the proximal end side. That is, the rigidities to bending of the elastic cylindrical portions 9, 9A, 9B increase along the direction from the distal end portion toward the proximal end portion of the insertion section 4.

Accordingly, also when the tightly wound coil 82 having the configuration as shown in Fig. 8 is used for the elastic cylindrical portion 9B, the part on the distal end side of the tightly wound coil 82 of the elastic cylindrical portion 9B is more easily bent than the part on the proximal end side of the tightly wound coil 2 of the elastic cylindrical portion 9B, as in Fig. 7.

As described above, according to the first and the second embodiments described above, an endoscope whose insertion section 4 has high insertion performance may be provided by making the insertion section 4 less prone to break by preventing the bending portion 8 of the insertion section 4 from being sharply bent.

Note that, in the two embodiments described above, the elastic cylindrical portions 9 and 9A are configured by including a tightly wound coil, but a tubular rubber member may also be used instead of the tightly wound coil.

Fig. 9 is a schematic cross-sectional diagram for describing a configuration of an elastic cylindrical portion having a rubber member, according to an example modification of the first and the second embodiments, the diagram showing an elastic cylindrical portion 9C along the axial direction. A circular cylindrical rubber member 83 is disposed between the proximal end portion of the coil receiving member 51 and the distal end portion of the coil receiving member 53.

The tubular rubber member 83 is flexible and elastic, and achieves the same effect as the tightly wound coil 52 described above.

Furthermore, the thickness of a thin portion of the rubber member 83 may be changed to increase stepwise along the way between the distal end side and the proximal end side as in the case shown in Fig. 7 where the wire diameter of a wire member is changed stepwise, or the thickness of the thin portion of the rubber member 83 may be changed to gradually increase from the distal end side toward the proximal end side as in the case shown in Fig. 8 where the wire diameter of a wire member is gradually changed.

Further note that the rigidities to bending at the distal end side and the proximal end side of an elastic cylindrical portion having a circular cylindrical shape may be changed by changing the material of the wire member in the case of a tightly wound coil and by changing the material of the rubber in the case of a rubber member. That is, the rigidity to bending of the elastic cylindrical portion may be changed by changing the wire diameter or the material of a wire member, or the material of a rubber member constituting the elastic cylindrical portion.

As described above, according to the two embodiments and the example modification described above, an endoscope having an insertion section with high insertion performance may be provided by making the insertion section less prone to break by preventing a bending portion of the insertion section from being sharply bent.

The present invention is not limited to the embodiments described above, and various changes and modifications may be made without departing from the spirit and scope of the present invention.

The present application claims benefit of Japanese Patent Application No. 2013-265326 filed in Japan on December 24, 2013, the entire contents of which are incorporated herein by reference.

**Claims**

1. An endoscope comprising:

    a rigid distal end portion provided in a distal end portion of an insertion section;
    a bending portion, provided on a proximal end side of the rigid distal end portion of the insertion section, and configured to bend according to a bending operation by an operation section;
    a flexible tube portion provided on a proximal end side of the bending portion; and
    an elastic cylindrical portion, provided between the bending portion and the flexible tube portion, and configured not to bend according to the bending operation by the operation section, the elastic cylindrical portion having a rigidity to bending higher than a rigidity to bending of the bending portion and lower than a rigidity to bending of the flexible tube portion.

**2.** The endoscope according to claim 1, wherein the rigidity to bending of the elastic cylindrical portion is not uniform along an axial direction of the insertion section.

**3.** The endoscope according to claim 1, wherein the rigidity to bending of the elastic cylindrical portion increases along a direction from the distal end portion toward a proximal end portion of the insertion section.

**4.** The endoscope according to claim 3, wherein the rigidity to bending of the elastic cylindrical portion is changed by changing a wire diameter or a material of a wire member constituting the elastic cylindrical portion.

**5.** The endoscope according to claim 3, wherein the rigidity to bending of the elastic cylindrical member increases stepwise along the direction from the distal end portion toward the proximal end portion of the insertion section.

**6.** The endoscope according to claim 1, wherein the elastic cylindrical portion includes a tightly wound coil.

**7.** The endoscope according to claim 6, wherein a distal end portion of the tightly wound coil is fixed to a first coil receiving member, and a proximal end portion of the tightly wound coil is fixed to a second coil receiving member.

**8.** The endoscope according to claim 7, wherein the first coil receiving member and the second coil receiving member have a cylindrical shape, and the distal end portion of the tightly wound coil is fitted with a step portion formed on a proximal end side of the first coil receiving member, and the proximal end portion of the tightly wound coil is fitted with a step portion formed on a distal end side of the second coil receiving member.

**9.** The endoscope according to claim 1, wherein the elastic cylindrical portion is a tubular rubber member.

**10.** The endoscope according to claim 1, comprising a coil pipe through which a bending wire configured to bend the bending portion is inserted, wherein a distal end portion of the coil pipe is fixed to a proximal end portion of the bending portion.

**11.** The endoscope according to claim 7, comprising a coil pipe through which a bending wire configured to bend the bending portion is inserted, wherein a distal end portion of the coil pipe is fixed to the first coil receiving member, and a proximal end side of the coil pipe is fixed to the second coil receiving member.

**12.** The endoscope according to claim 1, wherein an outer circumferential surface of the elastic cylindrical portion is covered with an elastic rubber tube.

**13.** The endoscope according to claim 7, wherein the elastic cylindrical portion, the first coil receiving member, and the second coil receiving member are covered with an elastic rubber tube.

**14.** The endoscope according to claim 6, wherein the tightly wound coil is not compressed in an axial direction of the insertion section.

**15.** The endoscope according to claim 3, wherein the rigidity to bending of the elastic cylindrical portion is changed by using a plurality of elastic cylindrical portions having wire members of different wire diameters or materials.

**16.** The endoscope according to claim 9, wherein a thickness of a thin portion of the rubber member increases along a direction toward the distal end portion or a proximal end portion of the insertion section.

**17.** The endoscope according to claim 9, wherein a thickness of a thin portion of the rubber member increases stepwise along a direction toward the distal end portion or a proximal end portion of the insertion section.

**18.** The endoscope according to claim 9, wherein a rigidity to bending of the rubber member is changed by changing a material of the rubber member.

**19.** The endoscope according to any one of claims 1 to 18, wherein a length of the elastic cylindrical portion along an axial direction of the insertion section is equal to or longer than a length of the bending portion along the axial direction of the insertion section.

# FIG. 1

EP 3 087 898 A1

# FIG. 2

# FIG. 3

# FIG. 4

# FIG. 5

# FIG. 6

# FIG. 7

EP 3 087 898 A1

# FIG. 8

# FIG. 9

## INTERNATIONAL SEARCH REPORT

| International application No. |
| --- |
| PCT/JP2014/077044 |

### A. CLASSIFICATION OF SUBJECT MATTER
*A61B1/00*(2006.01)i, *G02B23/24*(2006.01)i

According to International Patent Classification (IPC) or to both national classification and IPC

### B. FIELDS SEARCHED

Minimum documentation searched (classification system followed by classification symbols)
A61B1/00-1/32, G02B23/24-23/26

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 2006-218231 A (Olympus Corp.),<br>24 August 2006 (24.08.2006),<br>paragraphs [0047], [0130] to [0144]; fig. 30 to 32<br>& US 2010/0168519 A1 & EP 1849396 A1<br>& WO 2006/085620 A1 & KR 10-2007-0103445 A<br>& CN 101115432 A | 1-3,5,10,19<br>4,6-9,11-18 |
| Y | JP 11-56763 A (Olympus Optical Co., Ltd.),<br>02 March 1999 (02.03.1999),<br>paragraphs [0058] to [0064], [0080], [0084];<br>fig. 3<br>(Family: none) | 4,12,15 |

☒ Further documents are listed in the continuation of Box C.  ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 16 December 2014 (16.12.14) | 06 January 2015 (06.01.15) |

| Name and mailing address of the ISA/<br>Japan Patent Office | Authorized officer |
| --- | --- |
| Facsimile No. | Telephone No. |

Form PCT/ISA/210 (second sheet) (July 2009)

15

**INTERNATIONAL SEARCH REPORT**

International application No.

PCT/JP2014/077044

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2012-231886 A (Olympus Corp.), 29 November 2012 (29.11.2012), paragraphs [0030], [0079] & US 2013/0331651 A1 & EP 2664267 A1 & WO 2012/147443 A1 & CN 103402417 A | 6-8,11,13-14 |
| Y | JP 11-155871 A (Fuji Photo Optical Co., Ltd.), 15 June 1999 (15.06.1999), paragraphs [0006], [0012], [0015], [0016]; fig. 2 (Family: none) | 6-8,11,13-14 |
| Y | JP 2012-55569 A (Fujifilm Corp.), 22 March 2012 (22.03.2012), paragraph [0039] & CN 102397051 A | 6-8,11,13-14 |
| Y | JP 2001-333883 A (Asahi Optical Co., Ltd.), 04 December 2001 (04.12.2001), claim 19; paragraphs [0072] to [0110]; fig. 2 to 6 & US 2002/0010386 A1 & US 2004/0193013 A1 | 9,18 |
| Y | JP 2001-321324 A (Asahi Optical Co., Ltd.), 20 November 2001 (20.11.2001), paragraphs [0058] to [0105] & US 2002/0010386 A1 & US 2004/0193013 A1 | 16-17 |
| A | JP 2-131738 A (Olympus Optical Co., Ltd.), 21 May 1990 (21.05.1990), claim 1; fig. 2 (Family: none) | 1-19 |
| A | JP 2011-152361 A (Olympus Corp.), 11 August 2011 (11.08.2011), claim 6; paragraphs [0053], [0056], [0057] & WO 2011/092891 A1 | 1-19 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 1022641 U

- JP 2013265326 A